# EUROPÄISCHE PATENTSCHRIFT

(11) **EP 0 808 183 B1**
(45) Veröffentlichungstag und Bekanntmachung des Hinweises auf die Patenterteilung: **18.11.1998**
(21) Anmeldenummer: 96902862.0
(22) Anmeldetag: 09.02.1996
(51) Int. Cl.: A61M 1/10, A61M 1/12, F04B 43/02, F04B 9/04, F04B 9/02

(54) **ANTRIEBSVORRICHTUNG FÜR EIN KÜNSTLICHES HERZ**
ARTIFICIAL HEART DRIVING DEVICE
DISPOSITIF D'ENTRAINEMENT POUR COEUR ARTIFICIEL

(30) Priorität: 10.02.1995 DE 19505512
(43) Veröffentlichungstag der Anmeldung: 26.11.1997
(73) Patentinhaber: Bücherl, Sebastian, 14193 Berlin (DE); Frank, Jörn, 13435 Berlin (DE); Spiegelberg, Andreas, 21079 Hamburg (DE); Sauer, Igor Maximilian, 14057 Berlin (DE)
(72) Erfinder: Bücherl, Sebastian, 14193 Berlin (DE); Frank, Jörn, 13435 Berlin (DE); Spiegelberg, Andreas, 21079 Hamburg (DE); Sauer, Igor Maximilian, 14057 Berlin (DE)
(74) Vertreter: Pfenning, Meinig & Partner
(86) Internationale Anmeldenummer: DE9600236
(87) Internationale Veröffentlichungsnummer: WO9624394

(56) Entgegenhaltungen:
- EP-A- 0 146 445
- EP-A- 0 531 856
- US-A- 4 293 961
- US-A- 4 718 903

## Beschreibung

Die Erfindung betrifft eine Antriebsvorrichtung für ein künstliches Herz nach dem Oberbegriff des Hauptanspruchs.

Aus der europäischen Patentanmeldung 0 531 856 ist eine pulsierend arbeitende Blutpumpe bekannt, bei der in dem Blutpumpengehäuse mindestens ein von einem Kopplungspunkt auf einer dreieckigen geschlossenen Hypozykloidenbahn getriebener Kolben angeordnet ist, der eine Blutkammer periodisch zusammendrückt und wieder freigibt. Die eine Ecke der geschlossenen dreieckigen Hypozykloidenbahn weist in Richtung auf den Kolben, wodurch Kolbenbewegungen entstehen, die aus einer Vorhubphase, Rückhubphase und stationäre Hubphase entstehen. Diese bekannte Blutpumpe weist einen sehr komplexen Aufbau auf und besteht aus vielen Einzelteilen. Die Bewegung muß über eine große Anzahl von Lagern übertragen werden, wodurch viele Verluste entstehen. Aufgrund des komplexen Aufbaus ist die Lebensdauer des Antriebs dieser Pumpe nicht befriedigend.

US 4 718 903 beschreibt ein künstliches Herz, bei dem als Antrieb ein bewegbarer Körper verwendet wird, an dem Andruckplatten befestigt sind und der eine Rollbewegung ausführt. Der bewegbare Körper weist einen bidirektionalen Motor mit Motorgehäuse und eine Planetengetriebeanordnung auf, wobei letztere zwischen Motor und einer von zwei das Gehäuse abschließenden relativ zu diesem drehenden Abschlußteile derart angeordnet ist, daß Motor mit Gehäuse die durch Führungen geführte Rollbewegung ausführt und die Abschlußteile, die die Andruckplatten tragen, eine Gleitbewegung durchführen. Eine hin- und hergehende Bewegung wird durch Umpolen des Motors erzielt. Ein derartiges künstliches Herz ist aufgrund der Planetengetriebeanordnung aufwendig und weist viele drehende Teile auf. Darüber hinaus sind Totpunkte aufgrund der Bewegungsumkehr vorhanden und die Endlagen müssen für die Steuerung des Motors erkannt werden.

Der vorliegenden Erfindung liegt die Aufgabe zugrunde, eine Antriebsvorrichtung für ein künstliches Herz zu schaffen, die einen einfachen Aufbau aufweist und eine direkte Kraftübertragung gestattet, wobei die Standzeit erhöht werden soll.

Diese Aufgabe wird erfindungsgemäß durch die Merkmale der unabhängigen Ansprüche 1 und 12 gelöst.

Dadurch, daß der in eine Richtung drehende Motor direkt mit einem Antriebszahnrad verbunden ist und ein die Antriebsplatten betätigendes Bewegungselement mit Innenverzahnung oder Außenverzahnung direkt in Eingriff steht und bei Drehung des Zahnrades das Bewegungselement eine hin- und hergehende Bewegung ausübt, gibt es eine direkte Kraftübertragung von dem Motor über das Antriebszahnrad, das Bewegungselement auf die Andruckplatten. Aufgrund der direkten Kraftübertragung kann der Aufbau des Antriebes vereinfacht werden, wodurch seine Lebensdauer verlängert wird. Dies gilt gleichermaßen für die Lösung mit den Merkmalen des weiteren unabhängigen Anspruchs.

Durch die in den Unteransprüchen angegebenen Maßnahmen sind vorteilhafte Weiterbildungen und Verbesserungen möglich.

In vorteilhafter Weise ist das Bewegungselement als Innenzahnrad ausgebildet, das ein innenverzahntes Oval aufweist, wobei das innenverzahnte Oval mit dem Antriebszahnrad in Eingriff steht. Durch Drehung des Antriebszahnrades führt das Innenzahnrad eine hin- und hergehende Bewegung in Längsrichtung mit einer Versetzung senkrecht zur Längsrichtung durch, die direkt auf die Andruckplatten übertragen wird.

Durch die Führungsvorrichtung wird ein Eingriff zwischen dem Antriebszahnrad und dem Innenzahnrad in einer Zwangsführung sichergestellt.

Dadurch, daß an den Andruckplatten ein Gelkissen angeordnet ist, kann die Auf- und Abwärtsbewegung der Andruckplatten beim Richtungswechsel aufgenommen werden.

In einem weiteren bevorzugten Ausführungsbeispiel ist das Bewegungselement als Pendel ausgebildet, das an einem Ende gelenkig gelagert ist und an dem anderen Ende einen in der Nockenbahn ablaufenden Nockenfolger aufweist, wobei die Kolben gelenkig an senkrecht zu dem Pendel ausgebildeten Armen befestigt sind. Auch diese Ausführungsform liefert einen einfachen Aufbau und eine direkte Kraftübertragung der Antriebskraft des Motors auf die Kolben ohne große Verluste.

Ausführungsbeispiele der Erfindung sind in der Zeichnung dargestellt und werden in der nachfolgenden Beschreibung näher erläutert. Es zeigen:
- Fig. 1: schematische Ansichten eines ersten Ausführungsbeispiels der Erfindung, wobei Fig. 1a einen Schnitt längs der Schnittlinie A-A in Fig.1b, Fig. 1b eine Ansicht gemäß B-B in Fig. 1a, Fig. 1c eine Ansicht gemäß C-C in Fig. 1a und Fig. 1d eine Ansicht gemäß D-D nach Fig. 1a zeigen,
- Fig. 2: einen Bewegungsablauf des erfindungsgemäßen Antriebes,
- Fig. 3a bis 3d: eine schematische Schnittdarstellung des Antriebes nach einem zweiten Ausführungsbeispiel der Erfindung,
- Fign. 4a, 4b und 4c: schematische Schnittdarstellungen in Ebenen senkrecht zu der des Ausführungsbeispiels nach Fig. 3, wobei Fig. 4a der Fig. 4c entspricht, aber den Antrieb etwas genauer darstellt,
- Fig. 5a und 5b: schematische Schnittdarstellungen durch einen Antrieb nach einem dritten Ausführungsbeispiel der Erfindung,
- Fig. 6a und 6b: schematische Schnittdarstellungen durch einen Antrieb nach einem vierten Ausführungsbeispiel der Erfindung, und
- Fig. 7: eine schematische Schnittdarstellung durch einen Antrieb nach einem fünften Ausführungsbeispiel der Erfindung.

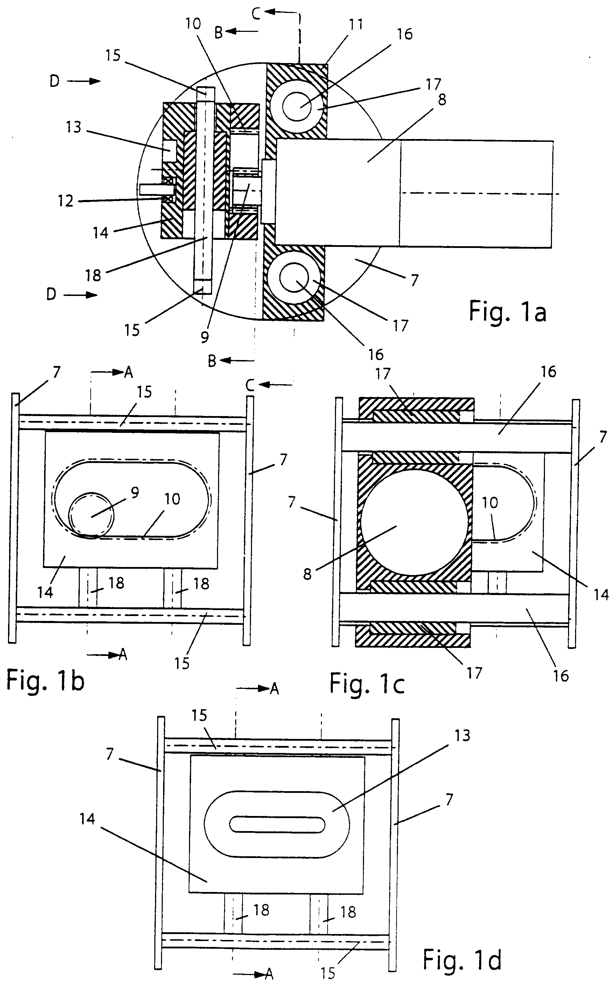

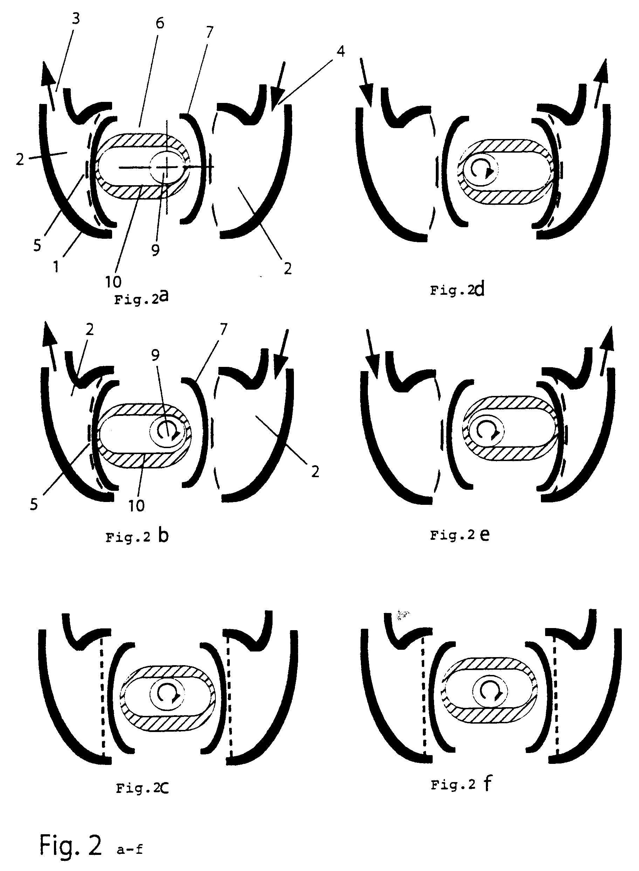

In den Fign. 1a bis 1d ist ein Ausführungsbeispiel eines Antriebs für ein künstliches Herz in verschiedenen Ansichten dargestellt und Fig. 2 zeigt eine prinzipielle Darstellung des Bewegungsablaufs des wesentlichen Teils des Antriebs in bezug auf eine Blutpumpe.

In Fig. 2 ist mit dem Bezugszeichen 1 das Pumpengehäuse angedeutet, das zwei Blutkammern 2 mit Blutein- und -auslässen 3, 4 einschließt. Die Blutkammern werden von Membranen 5, vorzugsweise aus Polyurethan, abgeschlossen, die entsprechend der Pumpbewegung bewegt werden. Zwischen den Membranen 5 ist ein Antrieb 6 vorgesehen, der auf Andruckplatten 7 wirkt, die zusammen mit den Membranen 5 die Kolben zur Veränderung des Volumens der Blutkammern 2 bilden.

Entsprechend Fig. 1a-d weist der Antrieb 6 einen Motor 8 auf, der als Gleichstrommotor ausgebildet sein kann und in eine Drehrichtung dreht und der an einem Gehäuse 11 oder Rahmen gelagert ist. Auf der Motorwelle sitzt ein Antriebszahnrad 9, das mit einem Innenzahnrad 10 in Eingriff steht. Das Innenzahnrad 10 weist ein innenverzahntes Oval auf, wobei die langen Seiten parallel zueinander liegen und jeweils durch Kreisbögen miteinander verbunden sind.

Um ständig eine formschlüssige Verbindung zwischen Antriebszahnrad 9 und der Innenverzahnung des Innenzahnrads 10 sicherzustellen, ist eine Führungsvorrichtung vorgesehen, die ein mit dem Gehäuse 11 verbundenes Kugellager 12 und eine Führungsbahn 13 umfaßt, in der das Kugellager 12 abläuft. Die Führungsbahn 13, die ovalförmig ausgebildet ist, ist in ihren Abmessungen an die Abmessungen des Antriebszahnrades 8 und des innenverzahnten Ovals angepaßt. Das Innenzahnrad 10 und die Führungsbahn 13 sind in einem Block 14 ausgeformt, der in der Figur als zwei fest miteinander verbundenen Teilen besteht.

Die Andruckplatten 7 sind über vier Längsstangen 15, 16 miteinander verbunden, von denen die parallelen Längsstangen 16 verschiebbar in mit dem Gehäuse 11 fest verbundenen Hülsen 17 geführt sind. Die Längsstangen 15 sind jeweils fest mit zwei Querstangen 18 verbunden, auf denen über Hülsen 19 der Block 14 mit dem Innenzahnrad 10 und der Führungsbahn 13 verschiebbar gelagert ist.

Die Funktionsweise des in Fig. 1 und Fig. 2 dargestellten Antriebs für das künstliche Herz ist wie folgt.

Das mit der Innenverzahnung des Innenzahnrads 10 in Eingriff stehende Antriebszahnrad 9 dreht sich beispielsweise entsprechend Fig. 2 im Uhrzeigersinn, wodurch sich der Block 14 mit dem Innenzahnrad 10 auf den Querstangen 18 verschiebt und gleichzeitig sich nach rechts bewegt. Dadurch werden die Andruckplatten 7 zusammen mit den Stangen 15,16,18 und dem Block 14 mit dem Innenzahnrad 10 gegen die Membran 5 nach rechts gedrückt, wodurch das Volumen der rechten Blutkammer 2 verringert wird und entsprechend Fig. 2d eine Entleerung stattfindet. Das Kugellager 12 läuft in der Führungsbahn 13 des Blocks 14 ab, wodurch der Eingriff zwischen den Zahnrädern 9, 10 sichergestellt wird. Beim Weiterdrehen des Zahnrades 9 entsprechend Fig. 1c schiebt sich der Block 14 mit dem Innenzahnrad 10 und der Führungsbahn wieder nach links solange, bis entsprechend Fig. 2a,b die Andruckplatte 7 das Volumen der linken Blutkammer auf das Minimum zusammendrückt, wobei die Entleerung in der linken Kammer 2 stattfindet, während die rechte Kammer gefüllt wird.

Wie in Fig. 2 rechts zu erkennen ist, verschiebt sich das Innenzahnrad 10 zusammen mit dem Block 14 auch nach oben und nach unten, wobei die senkrechte Bewegungsrichtung jeweils zum Zeitpunkt der Endsystole auftritt. Durch diese Richtungsverschiebung tritt eine Reibung zwischen der Andruckplatte 7 und der Membran 5 auf. Dies kann dadurch verhindert werden, daß ein Gelkissen zwischen der Andruckplatte 7 und Membran 5 vorgesehen wird, das auf der Andruckplatte angeordnet ist und die Bewegungen senkrecht zur Längsrichtung des Innenzahnrades aufnehmen und kompensieren kann. Es können auch andere Medien zur reibungs- und verschleißarmen Kraftübertragung vorgesehen werden.

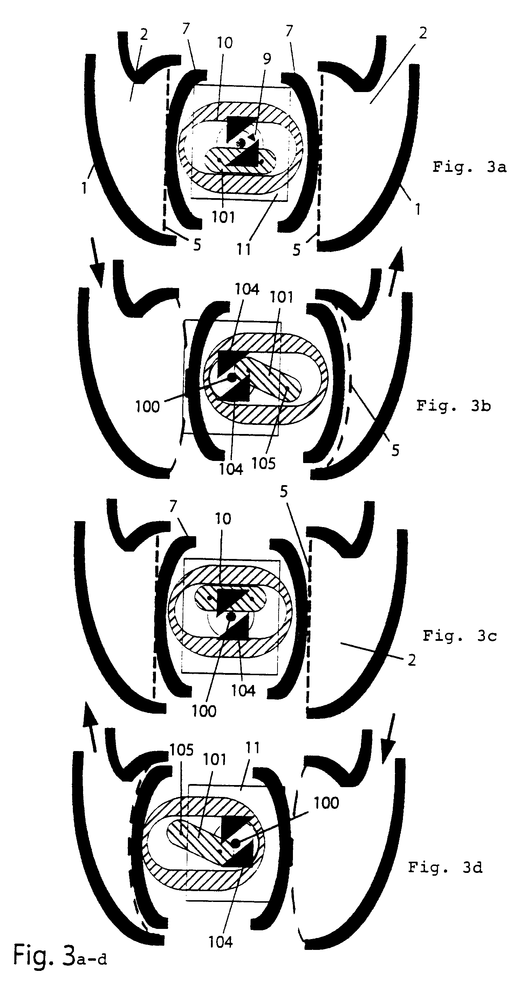

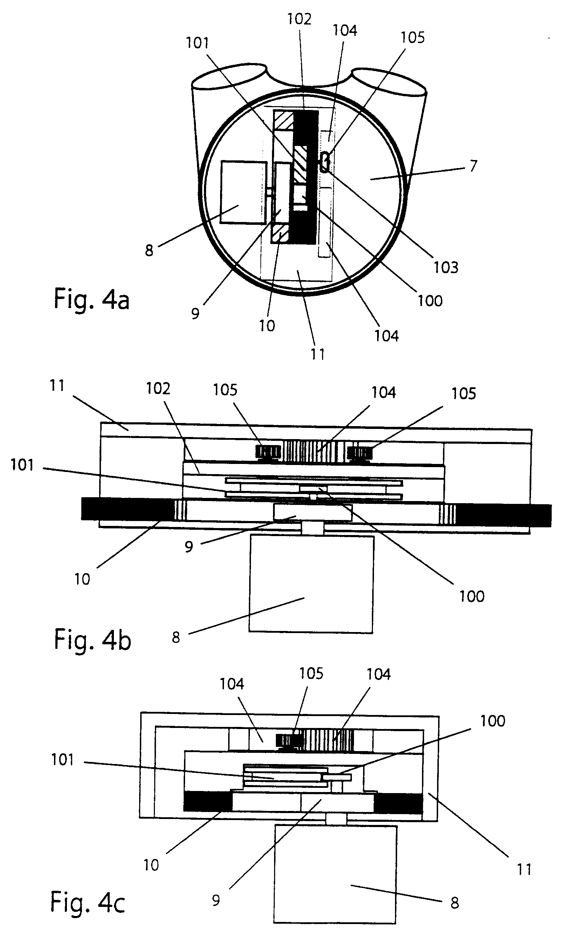

In Fig. 3 und Fig. 4 ist ein weiteres Ausführungsbeispiel des Antriebs eines künstlichen Herzens dargestellt, wobei in Fig. 3 der Bewegungsablauf dem nach Fig. 2 entspricht. Für gleiche Teile werden gleiche Bezugszeichen verwendet. In diesem Fall weist die Führungsvorrichtung, die die formschlüssige Verbindung zwischen Antriebszahnrad 9 und Innenverzahnung des Innenzahnrades 10 sicherstellt, eine auf dem Antriebszahnrad 9 bzw. Motorwelle sitzende Rolle 100 und ein Paßstück bzw. Paßstein 101 auf, die in ihren Abmessungen an die des Antriebszahnrades 8 und des Innenzahnrades 10 angepaßt sind. Das Paßstück 101 ist schwenkbar in bezug auf das Innenzahnrad 10 gelagert. Diese schwenkbare Lagerung kann, wie in Fig. 4 dargestellt ist, dadurch realisiert werden, daß ein Rahmen oder eine Abstützung 102 mit dem Innenzahnrad 10 fest verbunden ist, wobei in der Abstützung 102 gekrümmte Nuten vorgesehen sind. An dem Paßstück 101 sind Führungsstifte 103 angeordnet, die in der Abstützung vorgesehenen gekrümmten Nuten durchgreifen, die den Schwenkweg bzw. den Schwenkbereich des Paßstückes 101 entsprechend der Darstellung in Fig. 3 vorgeben, wobei der Übersichtlichkeit wegen die Abstützung 102 mit den gekrümmten Nuten in Fig. 3 nicht dargestellt ist. Selbstverständlich können die Führungsstifte auch durch Kugellager ersetzt werden, die in den entsprechenden Führungsnuten abrollen. An dem Gehäuse 11 als "feststehendes Teil", das den Motor 8 abstützt, sind eine obere und eine untere Ablenkschiene 104 angeordnet, und Rollen 105, die auf den Führungsstiften 103 sitzen, rollen auf den Ablenkschienen 104 entlang, um die Bewegung des Antriebszahnrades 9 bzw. des Innenzahnrades 10 im Bogenbereich zu unterstützen. Paßstück 101 und Innenzahnrad 10 sind so angeordnet, daß das Paßstück 101 in der Endsystole durch die Ablenkschienen zum Lagewechsel bezüglich des Innenovals gezwungen wird. Während das Antriebszahnrad 9 das Halbkreissegment des Innenovals durchläuft, kippt das Paßstück 101 auf die andere Breitseite des Ovals und gewährleistet so den Eingriff des Antriebszahnrades 9 und Innenzahnrades 10 beim Rücklauf des Ovals.

Ansonsten entspricht die Funktionsweise dieses Ausführungsbeispiels dem nach Fig. 1.

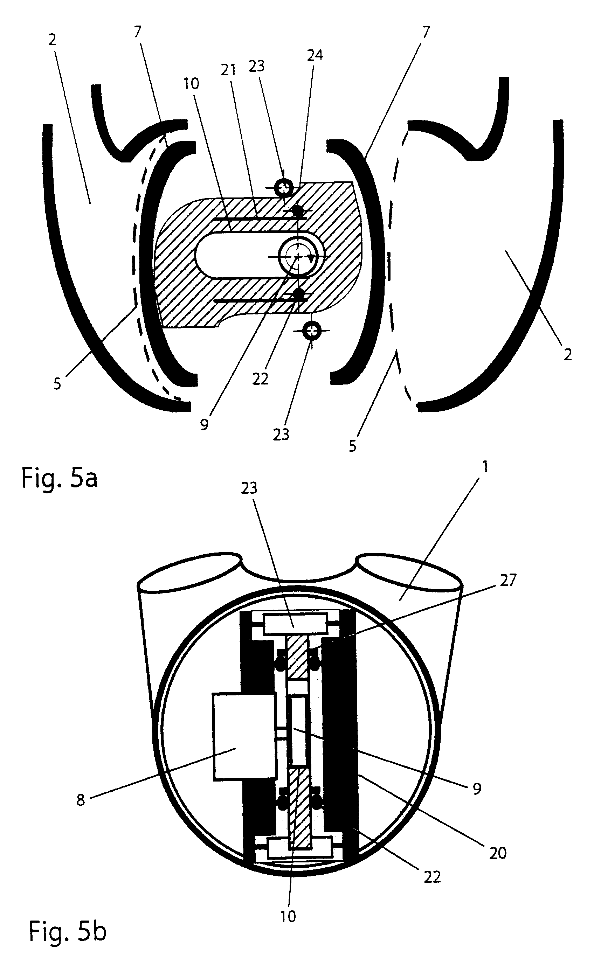

In Fign. 5a und 5b ist ein weiteres Ausführungsbeispiel dargestellt, bei dem die Zwangsführung des Antriebszahnrades 9 in bezug auf das Innenzahnrad 10 unterschiedlich ausgebildet ist. Auch hier werden gleiche Teile mit den gleichen Bezugszeichen entsprechend den Fign. 1 bis 4 verwendet. Wie aus Fig. 5b zu erkennen ist, ist ein Gehäuse 20 vorgesehen, das gleichfalls zur Aufnahme des Motors 8 dienen kann. Das Innenzahnrad 10 weist parallel zu den langen Seiten des innenverzahnten Ovals über und unter ihm jeweils eine Führungsschiene 21 auf jeder Seite auf. Diesen Führungsschienen 21 sind Rollen 22 zugeordnet, die auf mit dem Gehäuse 20 fest verbundenen Achsen angeordnet sind. Um die Umlenkbewegung im Bogenbereich des innenverzahnten Ovals zu unterstützen, sind Ablenkrollen 23 mit dem Gehäuse verbunden, die auf dem Umfang des Innenzahnrades 10 abrollen, auf dem jeweils zwei Nockenbahnen 24 vorgesehen sind.

Wie aus Fig. 5a zu erkennen ist, befindet sich das Innenzahnrad in einer Stellung, in der das Volumen der linken Blutkammer 2 verringert wird. Dabei laufen die mit dem Gehäuse verbundenen Rollen 22 oberhalb der Führungsschienen 21 an dem Innenzahnrad 10 ab. Die obere Ablenkrolle 23 läuft auf der oberen Nockenbahn 24 entlang und, um die Rollen 22 um die Führungsschienen 21 zu lenken und die Zwangsführung um den Bogenbereich sicherzustellen, ist die Nockenbahn 24 am Ende leicht gekrümmt derart ausgebildet, daß die Ablenkrolle 23 das Innenzahnrad 10 nach unten drückt, und im weiteren Verlauf der Bewegung schiebt sich das Innenzahnrad 10 langsam nach rechts, wobei die untere Nockenbahn 24 auf der unteren Rolle 23 abrollt und die Rollen 22, in Fig. 5a gesehen, unter den Führungsschienen 21 entlanglaufen.

In einem weiteren Ausführungsbeispiel können die Rollen 22 an dem Innenzahnrad 10 angeordnet sein und die Führungsschienen 21 am Gehäuse vorgesehen sein.

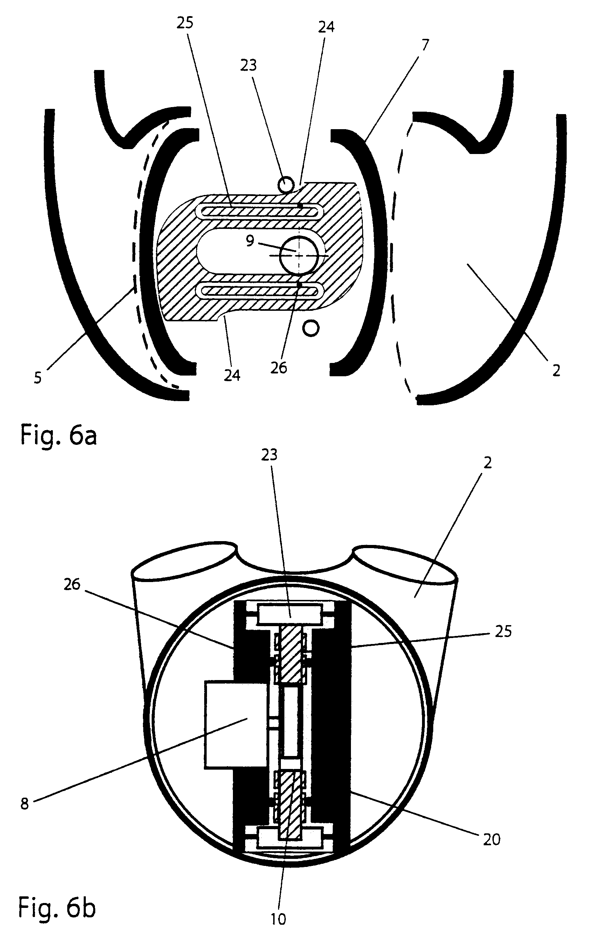

Fign. 6a und 6b zeigen ein ähnliches Ausführungsbeispiel wie Fign. 5a und 5b, wobei jedoch anstelle der Rollen 22 und der Führungsschienen 21 in dem Innenzahnrad 10 ober- und unterhalb des innenverzahnten Ovals durchgehende Führungsrinnen 25 entsprechend dem Weg des Antriebszahnrads 9 in dem innenverzahnten Oval vorgesehen sind. Entsprechende Führungsrinnen können in dem Gehäuse 20 angeordnet sein. Zwischen dem Gehäuse 20 und den Führungsrinnen 25 im Innenzahnrad laufen Kugeln 26, die zusammen mit den Ablenkrollen 23 und den Nockenbahnen 24 die Zwangsführung für das Innenzahnrad 10 bilden. Ansonsten ist die Funktionsweise wie in dem Ausführungsbeispiel nach Fign. 5a und 5b.

In den vorbeschriebenen Ausführungsbeispielen wurde das Innenzahnrad 10 als innenverzahntes Oval beschrieben. An dessen Stelle kann jedoch ein innenverzahntes Loch mit beliebiger, den gewünschten Bewegungen Vorgebende Umfangslinie verwendet werden.

In einem weiteren nicht dargestellten Ausführungsbeispiel kann ein Außenzahnrad mit Auflenverzahnung vorgesehen sein, wobei das Antriebszahnrad 9 auf der Außenverzahnung abläuft. Eine dem Innenzahnrad entsprechende Führungsvorrichtung stellt den Eingriff zwischen Außenverzahnung und Antriebszahnrad sicher.

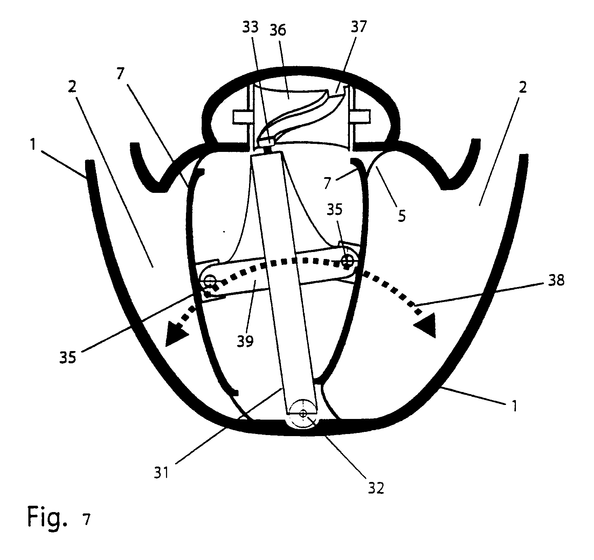

Fig. 7 zeigt ein weiteres Ausführungsbeispiel, bei dem das die Kolben eines Antriebs für das künstliche Herz betätigende Bewegungsorgan als Pendel 31 ausgebildet ist. Das stabförmige Pendel 31 ist an einem Ende in dem nicht näher dargestellten Pumpengehäuse 1 über ein Gelenk 32 schwenkbar gelagert. An dem anderen Ende ist ein als Walze ausgebildeter Nockenfolger 33 vorgesehen. Das stabförmige Pendel 31 ist mit seitlichen stabförmigen Auslegern 34 verbunden, an deren Enden über Verbindungsgelenke 35 die Andruckplatten 7 angeordnet sind. In diesem Ausführungsbeispiel ist die Membran 5 fest mit der Andruckplatte 7 verbunden. Der Nockenfolger 33 läuft in einer in einer Drehtrommel 36 mit integriertem Motor auf deren Umfang vorgesehenen Nockenbahn 37 ab, wobei die Nockenbahn 37 derart ausgebildet ist, daß bei einer Umdrehung der Trommel 36 durch die Zwangsführung des Nockenfolgers 33 in der Nockenbahn 37 das Pendel eine hin- und hergehende Bewegung entsprechend dem Pfeil 38 durchführt. Entsprechend den durch die Pendelbewegung ausgelösten Volumenänderungen wird die Blutkammer 2 gefüllt bzw. entleert.

## Patentansprüche

1. Antriebsvorrichtung für ein künstliches Herz mit in einem Pumpengehäuse angeordneten Blutkammern, die mittels von über einen Motor antreibbaren Andruckplatten zyklisch Blut ansaugen und ausstoßen, wobei der in eine Richtung drehende Motor (8) direkt mit einem Antriebszahnrad (9) verbunden ist und ein Bewegungselement mit Innen- oder Außenverzahnung (10;31,33) mit dem Antriebszahnrad (9) direkt in Zahneingriff steht und wobei bei Drehung des Antriebszahnrades (9) das Bewegungselement eine hin- und hergehende Bewegung ausübt und die mit ihm in Verbindung stehenden Andruckplatten unmittelbar betätigt.

2. Antriebsvorrichtung nach Anspruch 1, dadurch gekennzeichnet, daß das Bewegungselement ein Innenzahnrad aufweist, das mit dem Antriebszahnrad (9) in Eingriff ist und als innenverzahntes Loch mit vorgegebener Umfangslinie ausgebildet ist.

3. Antriebsvorrichtung nach Anspruch 2, dadurch gekennzeichnet, daß das Innenzahnrad als innenverzahntes Oval ausgebildet ist.

4. Antriebsvorrichtung nach einem der Ansprüche 1 bis 3, dadurch gekennzeichnet, daß die Andruckplatten (7) über Längs- und Querstangen (15,16,18) miteinander verbunden sind, daß auf den Querstangen ein das Innenzahnrad (10) aufweisender Block (14) verschiebbar geführt ist und daß mindestens eine Längsstange (16) verschiebbar in einem den Motor (8) aufnehmenden Gehäuse (11) gelagert ist.

5. Antriebsvorrichtung nach einem der Ansprüche 1 bis 4, dadurch gekennzeichnet, daß eine Führungsvorrichtung (12,13;100,101;21,22;24,25) vorgesehen ist, die den Eingriff zwischen dem Antriebszahnrad (9) und dem Innenzahnrad (10) oder Außenzahnrad sicherstellt.

6. Antriebsvorrichtung nach einem der Ansprüche 1 bis 5, dadurch gekennzeichnet, daß die Führungsvorrichtung als parallel zur Längsrichtung des innenverzahnten Lochs des Innenzahnrades (10) angeordnete Führungsschienen (21) und als auf den Führungsschienen (21) ablaufende Kugellager (22) ausgebildet sind, wobei die Führungsschienen (22) an dem feststehenden Gehäuse (20) und die Kugellager an dem Innenzahnrad (10) angeordnet sind oder umgekehrt.

7. Antriebsvorrichtung nach einem der Ansprüche 2 bis 5, dadurch gekennzeichnet, daß die Führungsvorrichtung als in dem Innenzahnrad (10) vorgesehene Führungsrinnen (13,26) ausgebildet sind, die jeweils eine Form entsprechend der Form des innenverzahnten Lochs aufweisen, wobei in den Führungsrinnen (13,25) mit den Gehäusen (11,20) in Verbindung stehende Rollelemente (12,26) abrollen.

8. Antriebsvorrichtung nach einem der Ansprüche 1 bis 5, dadurch gekennzeichnet, daß die Führungselemente als ovales Paßstück (101) und als auf der Achse des Antriebszahnrades (9) angeordnetes Kugellager (100) ausgebildet sind, wobei das Kugellager (100) auf dem äußeren Umfang des Paßstückes (101) abrollt und das Paßstück (101) schwenkbar mit dem Innenzahnrad (10) verbunden ist.

9. Antriebsvorrichtung nach einem der Ansprüche 1 bis 8, dadurch gekennzeichnet, daß die Führungsvorrichtung Ablenkelemente (104,23) aufweist, die eine Zwangsführung zwischen Antriebs- und Innenzahnrad (9,10) unterstützen.

10. Antriebsvorrichtung nach Anspruch 9, dadurch gekennzeichnet, daß die Ablenkelemente als mit dem Gehäuse verbundene Ablenkrollen (23) und als auf dem äußeren Umfang des Innenzahnrades (10) angeordnete Nockenbahnen (24) ausgebildet sind, wobei jeweils eine der Ablenkrollen (23) auf der ihr zugeordneten Nockenbahn (24) abrollt.

11. Antriebsvorrichtung nach Anspruch 9, dadurch gekennzeichnet, daß die Ablenkelemente als mit dem Gehäuse (11) verbundene Ablenkschienen (104) und als mit dem Paßstück (101) verbundene Ablaufrollen (105) ausgebildet sind.

12. Antriebsvorrichtung für ein künstliches Herz mit in einem Pumpengehäuse angeordneten Blutkammern, die mittels von über einen Motor antreibbaren Andruckplatten zyklisch Blut ansaugen und dieses ausstoßen, wobei der in eine Richtung drehende Motor (8) direkt mit einem Nockenelement (36,37) verbunden ist, mit dem ein pendelartiges Bewegungselement (10;31,33) in Eingriff steht, das bei Drehung des Nockenelments (36) eine hin- und hergehende Bewegung ausübt, wobei mit dem pendelartigen Bewegungselement die Andruckplatten verbunden sind.

13. Antriebsvorrichtung nach Anspruch 12, dadurch gekennzeichnet, daß das eine Ende des pendelartigen Bewegungselements (31) stationär gelenkig gelagert ist und das andere Ende einen Nockenfolger (33) aufweist, der auf dem als Nockenbahn (37) ausgebildeten Nockenelement (36) abläuft und eine Pendelbewegung bewirkt, und daß an dem Pendel (31) seitlich sich erstreckende Ausleger (39) angeordnet sind, die mit den Andruckplatten (7) gelenkig verbunden sind.

14. Antriebsvorrichtung nach Anspruch 12 oder 13, dadurch gekennzeichnet, daß die Nockenbahn (37) kontinuierlich auf der Umfangsfläche einer Trommel (37) ausgebildet ist, die von dem Motor angetrieben wird, wobei der Motor vorzugsweise in der Trommel integriert ist.

## Claims

1. Drive device for an artificial heart with blood chambers disposed in a pump housing, said chambers cyclically drawing in and expelling blood by means of a pressure plate drivable by a motor, the motor (8), rotating in one direction, being directly connected to a drive gear (9), and a movement component with internal or external gear teeth (10; 31, 33) is in direct meshing engagement with the drive gear (9), and upon rotation of the drive gear (9) executes a reciprocating movement, and directly actuates the pressure plates connected with it.

2. Drive device according to claim 1, characterised in that the movement component has an internal gearwheel which engages with the drive gear (9), and is in the form of an internally-geared hole with a predetermined peripheral line.

3. Drive device according to claim 2, characterised in that the internal geared wheel is in the form of an internally-geared oval.

4. Drive device according to one of claims 1 to 3, characterised in that the pressure plates (7) are interconnected via longitudinal and cross bars (15, 16, 18), in that a block (14) including the internal gearwheel (10) is displaceably guided on the cross bars, and in that at least one longitudinal rod (16) is displaceably mounted in a housing (11) accommodating the motor (8).

5. Drive device according to one of claims 1 to 4, characterised in that a guide device (12, 13; 100, 101; 21, 22; 24, 25) is provided, which ensures an engagement between the drive gear (9) and the internal geared wheel (10).

6. Drive device according to one of claims 1 to 5, characterised in that the guide device is designed as guide rails (21) disposed parallel to the longitudinal direction of the internally-geared aperture of the internal geared wheel (10), and as ball bearings (22) rolling on the guide rails (21), the guide rails (22) being disposed on the stationary housing (20) and the ball bearings on the internal geared wheel (10), or vice versa.

7. Drive device according to one of claims 2 to 5, characterised in that the guide device is designed as guide grooves (13, 26) provided in the internal geared wheel (10), each having a shape according to the shape of the internally-geared aperture, rolling members (12, 26) in connection with the housings (11, 20) rolling along the guide grooves (13, 25).

8. Drive device according to one of claims 1 to 5, characterised in that the guide members are in the form of an oval fitting pad (101), and as a ball bearing (100) disposed on the axis of the drive gear (9), the ball bearing (100) rolling on the external periphery of the fitting pad (101) and the fitting pad (101) being pivotally connected to the internal geared wheel (10).

9. Drive device according to one of claims 1 to 8, characterised in that the guide device has deflector members (104, 23), which reinforce a restricted guidance system between the drive gear and the internal geared wheel (9, 10).

10. Drive device according to claim 9, characterised in that the deflector members are designed as deflector rollers (23) connected to the housing, and as cam tracks (24) disposed on the external periphery of the internal geared wheel (10), one of the deflector rollers (23) respectively rolling along the cam track (24) associated therewith.

11. Drive device according to claim 9, characterised in that the deflector members are designed as deflector rails (104) connected to the housing (11), and as track rollers (105) connected to the fitting pad (101).

12. Drive device for an artificial heart with blood chambers disposed in a pump housing, said chambers cyclically drawing in and expelling blood by means of a pressure plate drivable by a motor, the motor (8), rotating in one direction, being directly connected to a cam member (36, 37) with which there engages a pendulum-like movement component (10; 31,33) which, upon rotation of the cam member (36) executes a reciprocating movement, the pressure plates being connected to the pendulum-like movement component.

13. Drive device according to claim 12, characterised in that one end of the pendulum-like movement component (31)is stationarily and pivotally mounted, and the other end has a cam follower (33), which rolls along the cam member (36) in the form of a cam track (37) and causes a pendulum movement, and in that there are disposed on the pendulum (31) laterally-extending extensions (39) which are pivotally connected to the pressure plates (7).

14. Drive device according to claim 12 or 13, characterised in that the cam track (37) is formed continuously on the circumferential surface of a drum (37) which is driven by the motor, the motor being preferably integrated in the drum.

## Revendications

1. Dispositif d'entraînement pour coeur artificiel comprenant des chambres de circulation du sang qui sont disposées dans un corps de pompe et qui, au moyen de plaques d'application de pression pouvant être entraînées par un moteur, aspirent et refoulent cycliquement le sang, le moteur (8) qui tourne dans un sens donné étant relié directement à une roue dentée d'entraînement (9), tandis qu'un élément de déplacement comportant une denture intérieure ou extérieure (10 ; 31, 33) engrène directement avec la roue dentée d'entraînement (9) et que, lorsque la roue d'entraînement (9) tourne, l'élément de déplacement exécute un mouvement dans un sens et dans l'autre et exerce directement une action sur les plaques d'application de pression qui sont solidaires de cet élément de déplacement.

2. Dispositif d'entraînement suivant la revendication 1, caractérisé en ce que l'élément de déplacement comporte une couronne à denture intérieure qui engrène avec la roue dentée d'entraînement (9) et est réalisée sous forme d'un évidement à denture intérieure présentant une ligne de contour préfixée.

3. Dispositif d'entraînement suivant la revendication 2, caractérisé en ce que la couronne à denture intérieure est réalisée sous forme d'une pièce ovale à denture intérieure.

4. Dispositif d'entraînement suivant l'une des revendications 1 à 3, caractérisé en ce que les plaques d'application de pression (7) sont reliées entre elles par des barres longitudinales et barres transversales (15, 16, 18), en ce qu'un bloc (14) comportant la couronne à denture intérieure (10) est guidé d'une manière coulissante sur les barres transversales et en ce qu'au moins une barre longitudinale (16) est montée coulissante dans un boîtier (11) servant à loger le moteur (8).

5. Dispositif d'entraînement suivant l'une des revendications 1 à 4, caractérisé à ce qu'il est prévu un dispositif de guidage (12, 13 ; 100, 101 ; 21, 22 ; 24, 25) qui assure l'engrènement entre la roue dentée d'entraînement (9) et la couronne à denture intérieure (10) ou roue à denture extérieure.

6. Dispositif d'entraînement suivant l'une des revendications 1 à 5, caractérisé en ce que le dispositif de guidage est réalisé sous forme de rails de guidage (21), disposés parallèlement à la direction longitudinale de l'évidement à denture intérieure de la couronne à denture intérieure (10), et de roulements à billes (22) se déplaçant sur les rails de guidage (21), les rails de guidage (22) étant disposés sur le boîtier fixe (20) et les roulements à billes sur la couronne à denture intérieure (10) ou vice versa.

7. Dispositif d'entraînement suivant l'une des revendications 2 à 5, caractérisé en ce que le dispositif de guidage est réalisé sous forme de gorges de guidage (13, 25) qui sont prévues dans la couronne à denture intérieure (10) et qui ont chacune une forme correspondant à la forme de l'évidement à denture intérieure, tandis que des éléments de roulement (12, 26) reliés au boîtier (11, 20) roulent dans les gorges de guidage (13, 25).

8. Dispositif d'entraînement suivant l'une des revendications 1 à 5, caractérisé en ce que le dispositif de guidage est réalisé sous forme d'une pièce d'adaptation (101), de forme ovale, et d'un roulement à billes (100) disposé sur l'axe de la roue dentée d'entraînement (9), le roulement à billes (100) roulant sur la surface périphérique extérieure de la pièce d'adaptation (101) et la pièce d'adaptation (101) étant reliée d'une manière basculante à la couronne à denture intérieure (10).

9. Dispositif d'entraînement suivant l'une des revendications 1 à 8, caractérisé en ce que le dispositif de guidage comprend des éléments déflecteurs (104, 23) qui contribuent à un guidage obligatoire entre la roue dentée d'entraînement et la couronne à denture intérieure (9, 10).

10. Dispositif d'entraînement suivant la revendication 9, caractérisé en ce que les éléments déflecteurs sont réalisés sous forme de galets déflecteurs (23), reliés au boîtier, et de chemins de came (24) disposés sur la surface périphérique extérieure de la couronne à denture intérieure (10), chacun des galets déflecteurs (23) roulant respectivement sur le chemin de came (24) qui lui est associé.

11. Dispositif d'entraînement suivant la revendication 9, caractérisé en ce que les éléments déflecteurs sont réalisés sous forme de rails déflecteurs (104), reliés au boîtier (11), et de galets de roulement (105) reliés à la pièce d'adaptation (101).

12. Dispositif d'entraînement pour coeur artificiel comprenant des chambres de circulation du sang qui sont disposées dans un corps de pompe et qui, au moyen de plaques d'application de pression pouvant être entraînées par un moteur, aspirent et refoulent cycliquement le sang, le moteur (8) qui tourne dans un sens donné étant relié directement à un élément de came (36, 37) avec lequel vient en prise un élément de déplacement (10 ; 31, 33), analogue à un bras pendulaire, qui exécute un mouvement dans un sens et dans l'autre lorsque l'élément de came (36) tourne, les plaques d'application de pression étant reliées à l'élément de déplacement analogue à un bras pendulaire.

13. Dispositif d'entraînement suivant la revendication 12, caractérisé en ce qu'une première extrémité de l'élément de déplacement (31) analogue à un bras pendulaire est montée articulée en un emplacement fixe et l'autre extrémité comporte un galet suiveur de came (33) qui se déplace sur l'élément de came (36), réalisé sous forme d'un chemin de came (37), et exécute un mouvement pendulaire et en ce qu'il est prévu, disposés sur l'élément pendulaire (31), des bras en porte-à-faux qui s'étendent transversalement et sont reliés d'une manière articulée aux plaques d'application de pression (7).

14. Dispositif d'entraînement suivant la revendication 12 ou 13, caractérisé en ce que le chemin de came (37) est réalisé d'une manière continue sur la surface périphérique d'un tambour (37) qui est entraîné par le moteur, le moteur étant de préférence intégré dans le tambour.
